# EUROPÄISCHE PATENTANMELDUNG

(11) **EP 0 640 615 A1**
(43) Veröffentlichungstag der Anmeldung: **01.03.1995**
(21) Anmeldenummer: 94112384.6
(22) Anmeldetag: 08.08.1994
(51) Int. Cl.: C07H 23/00, C07C 259/06, A61K 31/71, C12P 19/26, C12N 1/20

(54) **Chelatoren, ihre Herstellung aus den Antibiotika Salmycin A, B, C oder D und deren Verwendung**

(30) Priorität: 13.08.1993 DE 4327226
(71) Anmelder: HOECHST AKTIENGESELLSCHAFT, D-65929 Frankfurt am Main (DE)
(72) Erfinder: Vertesy, Lazlo, Dr., D-65817 Eppstein (DE); Aretz, Werner, Dr., D-61462 Königstein (DE); Fehlhaber, Hans-Wolfram, Dr., D-65510 Idstein (DE)

(57) **Zusammenfassung**

Aus den Antibiotika Salmycin A, B, C und D, die durch Fermentation gewonnen werden, werden die neuen Stoffe Nordanoxamin, Desferri-Nordanoxamin, Desferri-Salmycin A, B, C und D sowie die bekannten Stoffe Danoxamin und Desferri-Danoxamin hergestellt. Die Stoffe werden als Chelatoren und Arzneimittel verwendet.

## Beschreibung

Gegenstand der vorliegenden Erfindung sind Chelatoren, deren Herstellung aus den Salmycinen A, B, C oder D und ihre Verwendung als Arzneimittel.

Salmycine können durch Fermentation mit Hilfe des Mikroorganismus Streptomyces violaceus 37290 (DSM 8286) gewonnen werden (Patent Anmeldung: L. Vertesy et al., "Antibiotics, called Salmycin A, B and C, a process for their preparation and their use as a pharmaceutical", Hoechst AG (vgl. auch EP 93 118 511.9)). Die Salmycine sind eisenhaltige Antibiotika, sogenannte Sideromycine, die aus einer Chelat- und einer Amino-Disaccharid-Komponente bestehen. Die vermuteten Strukturen der Salmycin A bis D werden durch Formel I dargestellt. 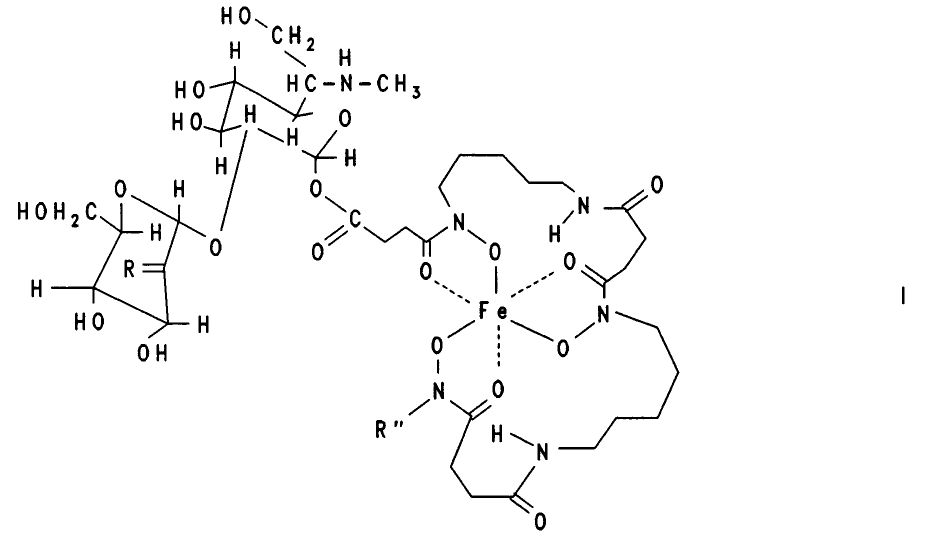
- Salmycin A:: R = NOH, R'' = -(CH₂)₅-OH
- Salmycin B:: R = O, R'' = -(CH₂)₅-OH
- Salmycin C:: R = O, R'' = -(CH₂)₄-OH
- Salmycin D:: R = NOH, R'' = -(CH₂)₄-OH
Aus Salmycin A und B wird durch Hydrolyse das bereits bekannte Eisenchelat Danoxamin (P. Huber et al., Helv. Chim. Acta **69** (1986) 236-245) hergestellt. Danoxamin hat die Summenformel C₂₇H₄₆FeN₅O₁₁ und ein Molekulargewicht von 672 g/mol. Formel II zeigt die Struktur von Danoxamin. 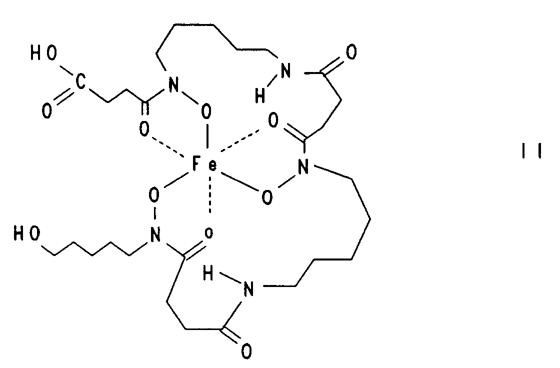
Überraschenderweise wurde nun gefunden, daß aus den Salmycinen C und D das bisher nicht beschriebene Eisenchelat Nordanoxamin gewonnen werden kann.

Erfindungsgegenstand ist demzufolge die Verbindung mit der Summenformel C₂₆H₄₄FeN₅O₁₁ mit einem Molekulargewicht von 658 g/mol, genannt Nordanoxamin, sowie dessen eisenfreie Form mit der Summenformel C₂₆H₄₇N₅O₁₁ und einem Molekulargewicht 605 g/mol, genannt Desferri-Nordanoxamin. Nordanoxamin hat die folgende Struktur 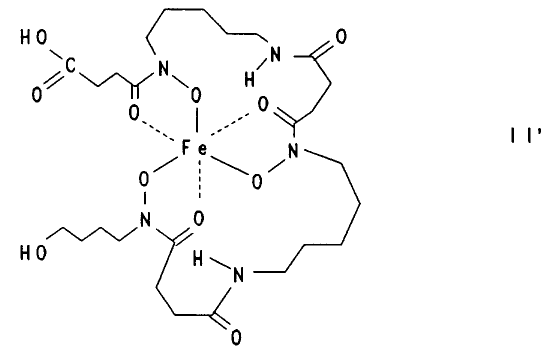
Ein weiterer Gegenstand der vorliegenden Erfindung sind die eisenfreien Verbindungen Desferri-Salmycin A, B, C und D.

Weiterhin gehört zum Gegenstand der vorliegenden Erfindung ein Verfahren zur Herstellung von Danoxamin und Nordanoxamin durch Hydrolyse von Salmycin A, B, C oder D.

Die Salmycine stammen vorzugsweise aus einem fermentativen Prozess mit dem Mikroorganismus Streptomyces violaceus (DSM Nr. 8286) wie in der Patentanmeldung von L. Vertesy et al., "Antibiotics, called Salmycin A, B and C, a process for their preparation and their use as a pharmaceutical" und EP 93 118 511.0 beschrieben ist. Die Hydrolyse der Salmycine sollte unter milden Bedingungen ausgeführt werden und kann in verdünnter Lauge, z.B. Ammoniumhydroxid-Lösung, bei Raumtemperatur erfolgen, vorzugsweise bei pH 9,5. Im allgemeinen ist die Hydrolyse der Salmycine nach 2 Stunden weitgehend abgeschlossen. Es ist vorteilhaft, die Reaktionslösung für die weitere Aufarbeitung zu neutralisieren. Das enthaltene Danoxamin oder Nordanoxamin kann durch chromatographische Methoden, insbesondere Umkehrphasen-Chromatographie, z.B. mit C₁₈-Kieselgel als Trägermaterial und einem Gemisch aus Wasser und Acetonitril als Fließmittel, vorzugsweise einem Konzentrationsgradienten von 0-15 Vol.-% Acetonitril, abgetrennt und gereinigt werden.

Die Herstellung von Danoxamin, Nordanoxamin und deren eisenfreien Formen aus den Salmycinen A, B, C bzw. D ist besonders vorteilhaft gegenüber bekannten Verfahren, da die Produkte in größeren Mengen zugänglich sind und die Gewinnung besonders wirtschaftlich erfolgen kann. Danoxamin konnte bisher nicht technisch verwendet werden, da es bisher nur in sehr geringen Mengen aus dem schwer zugänglichen Danomycin hergestellt werden konnte. Außerdem können die Produkte erfindungsgemäß in hoher Reinheit erhalten werden.

Zum Gegenstand der vorliegenden Erfindung gehört weiterhin ein Verfahren zur Herstellung von Desferri-Danoxamin aus Salmycin A und/oder B und Desferri-Nordanoxamin aus Salmycin C und/oder D durch Hydrolyse und Behandlung mit anderen Komplexbildnern oder durch Reduktion des gebundenen Fe³⁺-Ions. Die auf diese Weise gewonnenen Verbindungen sind sehr wirksam in der Bindung der freien Ionen Fe³⁺ und Al³⁺. Man erhält auf diese Weise das bereits beschriebene Desferri-Danoxamin (P. Huber et al., Helv. Chim. Acta **69** (1986) 236-245) und das bisher nicht bekannte Desferri-Nordanoxamin, das die Summenformel C₂₆H₄₇N₅O₁₁ und ein Molekulargewicht 605 g/mol hat.

Zur Herstellung der eisenfreien Formen Desferri-Danoxamin oder Desferri-Nordanoxamin kann auch auf eine Isolierung von Danoxamin und Nordanoxamin verzichtet werden. Der Reaktionsansatz kann nach erfolgter Hydrolyse direkt zur Entfernung des gebundenen Eisens mit einem Komplexbildner, einem eisenbindenden Reagenz oder einem Reduktionmittel versetzt werden oder einer kathodischen Reduktion unterzogen werden. Als Komplexbildner eignen sich alle gebräuchlichen Komplexierungsmittels, die in der Lage sind, Eisen-III-Ionen zu binden, wie z.B. Ethylendiamintetraessigsäure oder 8-Hydroxychinolin. Schwefelwasserstoff ist ein Beispiel für ein eisenbindendes Reagenz. Als Reduktionsmittel können dienen z.B. Natrium-Thiosulfat, Natriumsulfit oder Hydrochinon. Eine kathodische Reduktion erfolgt vorzugsweise in geteilter Zelle mit einer Kathode aus Platin, Nickel, Stahl, Graphit, Glaskohlenstoff oder anderen üblichen Kathodenmaterialien.

Weiterhin gehört zum Gegenstand der vorliegenden Erfindung ein Verfahren zur Herstellung von Desferri-Salmycin A, Desferri-Salmycin B, Desferri-Salmycin C und Desferri-Salmycin D durch Behandlung von Salmycin A, B, C oder D mit komplexierenden oder eisenbindenden Reagenzien oder durch Reduktion des gebundenen Fe³⁺-Ions. Die bevorzugten Herstellungsbedingungen sind bereits oben für die Herstellung von Desferri-Danoxamin und Desferri-Nordanoxamin beschrieben.

Die eisenfreien Verbindungen Desferri-Danoxamin, Desferri-Nordanoxamin, Desferri-Salmycin A, Desferri-Salmycin B, Desferri-Salmycin C und Desferri-Salmycin D dienen als Chelatoren von dreiwertigen Metallionen, insbesondere der Metallionen Fe³⁺ und Al³⁺. Aufgrund dieser Eigenschaften gibt es für diese Verbindungen vielfältige technische Verwendungen. Besondere Bedeutung haben Sie in der Pharmazie. In vivo verabreicht nehmen diese Chelatoren im Organismus Fe³⁺ und Al³⁺ auf und fördern damit die Ausscheidung dieser Ionen. Dadurch stellen sie wertvolle Arzneimittel dar, die insbesondere bei der Hämochromatose, einer Eisenspeicherkrankheit mit zu hohen Fe³⁺-Konzentrationen, oder bei der Alzheimerschen Krankheit mit zu hohen Al³⁺-Konzentrationen mit Erfolg eingesetzt werden können. Gegenüber Desferrioxamin B (s. B.E. Roggo u. H.H. Peter, J. Antibiotics **46** (1993) 294-299), das als Arzneimittel bereits auf dem Markt ist (®Desferal), weisen Desferri-Danoxamin und Desferri-Nordanoxamin keine eingeschränkte Verträglichkeit auf und sind selbst in hohen Dosen untoxisch. Desferri-Salmycin A, B, C und D haben ähnliche Eigenschaften. Ein weiterer Gegenstand der vorliegenden Erfindung ist deshalb die Verwendung von Desferri-Danoxamin, Desferri-Nordanoxamin, Desferri-Salmycin A, B, C und D sowie deren physiologisch verträgliche Salze, wie z.B. Lithium-, Natrium-, Kalium-, Calzium- oder Ammoniumsalz als Arzneimittel.

Die erfindungsgemäßen Arzneimittel können ein oder mehrere der aufgeführten Chelatoren und einen oder mehrere Träger- oder Hilfsstoffen wie zum Beispiel Füllmittel, Emulgatoren, Gleitmittel, Geschmackstoffe, Farbstoffe oder Puffersubstanzen enthalten und können in einer geeigneten pharmazeutischen Form wie beispielsweise Tabletten, Kapseln, eine Suspension oder Lösung parenteral verabreicht werden.

Beispiele für Trägerstoffe sind Tragacanth, Laktose, Talk, Agar-Agar, Polyglykole, Hydrokolloide, Ethanol oder Wasser. Geeignet und bevorzugt für eine parenterale Verabreichung sind z.B. Suspensionen oder Lösungen in Wasser. Es ist ebenfalls möglich, die aktive Substanz ohne Zusatzstoffe in geeigneter Form wie Kapseln zu verabreichen.

### Beispiele

### Beispiel 1: Anzucht von Streptomyces violaceus

Streptomyces violaceus (DSM 8286) wird auf folgendem festen Nährboden gehalten:

| | |
|---|---|
| Stärke: | 10 g/l |
| Casein: | 1 g/l |
| Pepton: | 1 g/l |
| Hefeextrakt: | 1 g/l |
| Malzextrakt: | 10 g/l |
| K₂HPO₄: | 0,5 g/l |
| Agar: | 15 g/l |

Das Medium wird für 30 Minuten bei 121 °C sterilisiert, auf 45 °C gekühlt und in Petrischalen abgefüllt. Die Petrischalen werden mit einer Sporensuspension beimpft und 8 Tage bei 28 °C bebrütet. Die Lagerung erfolgt bei 4 °C.
1 cm² einer bebrüteten Platte dient als Inokulum von 500 ml Vorkultur folgender Zusammensetzung:

| | |
|---|---|
| Glucose: | 15 g/l |
| Sojamehl: | 15 g/l |
| Cornsteep, fl. | 5 ml/l |
| CaCO₃: | 2 g/l |
| NaCl: | 5 g/l |
| pH 7,2 | |

Die Inkubation erfolgt für 2 Tage bei 28 °C auf einem Rotationsschüttelgerät bei einer Schüttelfrequenz von 240 Upm. 25 ml der Vorkultur dienen als Inokulum einer Hauptkultur (50 ml/ 300 ml Erlenmeyerkolben) folgender Zusammensetzung:

| | |
|---|---|
| Sojamehl: | 20 g/l |
| Mannit: | 20 g/l |
| pH 7,5 | |

Die Hauptkultur wird 3-4 Tage bei 28 °C und 240 Upm bebrütet. Nach dieser Zeit wird im Diffusionstest eine biologische Aktivität von 21-23 mm Hemmhofdurchmesser gegen Staphylococcus aureus 209 P festgestellt.

### Beispiel 2: Produktion der Salmycine A, B, C und D im Fermenter

Analog Beispiel 1 erfolgt Stammhaltung und Vorkulturanzucht von Streptomyces violaceus (DSM 8286). Die Hauptkultur erfolgt in einem 12 l-Fermenter mit 9 l Hauptkulturnährlösung aus Beispiel 1. Das Inokulum beträgt 1 % des Fermentervolumens. Die Bebrütung erfolgt für 2-3 Tage bei 28 °C, 500 Upm und einer Belüftungsrate von 0,5 l/min.
Im Durchschnitt werden biologische Aktivitäten von 22-27 mm Hemmhof gegen Staphylococcus aureus 209 P erzielt.

### Beispiel 3: Isolierung von Roh-Salmycin

180 l Filtrat der nach Beispiel 2 gewonnenen Kulturen werden auf pH 6,8 eingestellt und auf eine mit 17 l eines Adsorptionsharzes gefüllte Säule aufgetragen. Als Adsorbens wird ein Styrol/Divinylbenzol-Copolymer in Pulverform wie ®Diaion HP-20 (Mitsubishi Chem. Ind., Japan) bevorzugt verwendet. Danach wäscht man den beladenen Träger mit entsalztem Wasser. Der Salmycin-Komplex wird nun mit einem 0-20 Vol.-% Isopropanol enthaltenden Gradienten eluiert. Fraktionen werden gesammelt und auf antibakterielle Wirksamkeit untersucht. Die Salmycin-haltigen Fraktionen werden vereinigt (ca. 30 l insgesamt). Die Flüssigkeit wird zur Entfernung von sauren Verunreinigungen über eine Anionenaustauscher-Säule (z.B. 1 l Diethylaminoethyl-Sepharose wie DEAE-®Sephadex Fast Flow-Ionenaustauscher von Pharmacia Fine Chemicals AB, Schweden) filtriert, die vorher mit Ammoniumacetat-Lösung, pH 7,0, äquilibriert worden ist. Anschließend wird die Lösung durch Ultrafiltration mit einer semipermeablen und permselektiven Membran wie z.B. einer "®Nadir UF-CA-1"-Membran (Hoechst; Frankfurt, Deutschland) konzentriert und gefriergetrocknet. Es resultieren 38 g des Salmycin-Rohproduktes.

### Beispiel 4: Vor-Trennung des Rohproduktgemisches

35 g des nach Beispiel 3 gewonnen Rohproduktgemisches werden in 500 ml destilliertem Wasser gelöst und auf eine 3,5 l fassende Säule (11,3 cm innerer Durchmesser, 36 cm Höhe) aufgegeben, die mit einem Adsorbens auf Styrol/Divinylbenzol-Copolymer-Basis wie z.B. "®MCI GEL CHP 20 P" (Mitsubishi Kasei Corp., Tokyo, Japan) gepackt ist. Es wird mit Wasser nachgewaschen und mit einem Wasser/10 Vol.-% Isopropanol in Wasser-Gradienten eluiert. Fraktioniert wird literweise. Fraktionen mit Salmycin B und C und Fraktionen, die hauptsächlich Salmycin A und D enthalten, werden erhalten. Konzentrierung im Vakuum und Gefriertrocknung ergeben 3,2 g Rohgemisch von Salmycin B und C sowie 1,4 g Roh-Salmycin A und D.

### Beispiel 5: Reinigung des Salmycin B und C

Das nach Beispiel 4 erhaltene Rohgemisch von Salmycin B und C wird über eine mit Molekularsieb gefüllte Säule (10 cm innerer Durchmesser, 52 cm Höhe) gegeben. Die Säulenpackung besteht z.B. aus 4 l ®Fractogel TSK HW-40 F (E. Merck, Darmstadt, Deutschland), einem hydrophilen Vinyl-Polymer für die Gelpermeationschromatographie für die Trennung von niedermolekularen Verbindungen wie Dextranen im Molekulargewichtsbereich 100-7000 g/mol. Als Fließmittel dient ein Gemisch aus Wasser und Methanol (1:1) mit einem Zusatz von 1 Vol.-% Essigsäure. Fraktionen, die Salmycin B oder C enthalten, werden vereinigt und gefriergetrocknet. Man erhält 510 mg eines Rohprodukts, das 82 Gew.-% Salmycin B und 3 Gew.-% Salmycin C enthält.

### Beispiel 6: Reinigung der Salmycine A und D

1,4 g Roh-Salmycin A und D werden mittels einer mit Molekularsieb gefüllten Säule gereinigt, wie in Beispiel 5 beschrieben. Das Fließmittel besteht aus vier Teilen Wasser und einem Teil Ethanol. Die Fraktionen, die Salmycin A und D enthalten, werden vereinigt. Konzentrierung und Getriertrocknung ergeben 240 mg eines Rohproduktes mit 85 Gew.-% Salmycin A und D.

### Beispiel 7: Reingewinnung der Salmycine A und D

Die nach Beispiel 6 gewonnenen Stoff (240 mg) werden in destilliertem Wasser gelöst und durch Umkehrphasen-Chromatographie gereinigt. Eine Säule mit 3,2 cm innerem Durchmesser und 25 cm Höhe ist mit 200 ml C₁₈-Kieselgel wie z.B. ®Nucleosil 12 C₁₈AB (Macherey & Nagel, Düren, Deutschland) gepackt. Als Fließmittel dient ein Wasser-Acetonitril-Gradient (0-10 Vol.-% Acetonitril). 25 ml-Fraktionen werden gesammelt. Fraktionen, die Salmycin D bzw. A enthalten, werden vereinigt. Konzentrierung und Gefriertrocknung ergeben 130 mg Salmycin A (99 Gew.-%) sowie ca. 1,5 mg Salmycin D.

### Charakterisierung von Salmycin A:

a) Hochauflösende FAB-Massenspektometrie:
   Molekülionen-Peak von M + H⁺ : 1053,4050 ± 0,0006 Dalton.
   Masse (berechnet für M + H⁺ , monoisotopisch): 1053,4052 Dalton.
   Formel: C₄₁H₇₀FeN₇O₂₁
b) ¹H-NMR: Signal bei 2,8 ppm in D₂O (N-Methyl-Gruppe), keine weiteren Signale für Methyl-Protonen.
c) UV-vis der wäßrigen Lösung (Phosphat-puffer, pH 7,0):
   Endabsorption und breite Absorption um 430 nm (log ε = 3,3).

### Charakterisierungvon Salmycin D:

a) ESI-MS:
   Molekülionen-Peak M + H⁺ : 1039,8 Dalton
   Formel: C₄₀H₆₈FeN₇O₂₁
b) UV-vis der wäßrigen Lösung (Phosphat-Puffer, pH 7,0):
   Endabsorption und breite Absorption um 430 nm (log ε = 3,3).

### Beispiel 8: Reingewinnung des Salmycin B und C

Das nach Beispiel 5 gewonnene Gemisch aus Salmycin B und C wird wie in Beispiel 7 durch Umkehrphasen-Chromatographie gereinigt. Das Fließmittel besteht aus einem Gemisch von Wasser, 6 Vol.-% Acetontril und 0,1 Vol.-% Trifluoressigsäure. 402 mg Salmycin B und 8 mg Salmycin C werden erhalten.

### Charakterisierung von Salmycin B:

a) Hochauflösende FAB-Massenspektrometrie:
   Molekülionen-Peak von M + H⁺ : 1038.3941 ± 0.0007 Dalton
   Molekülionen-Peak von M + H⁺ + H₂O: 1056.406 ± 0.001 Dalton
   Masse berechnet für M + H⁺ + H₂O: 1056.4053 Dalton
   Formel: C₄₁H₆₉FeN₆O₂₁
b) ¹H-NMR: Signal bei 2,8 ppm in D₂O (N-Methyl-Gruppe), keine weiteren Signale für Methyl-Protonen.
c) UV-vis der wäßrigen Lösung (Phosphat-Puffer, pH 7,0):
   Endabsorption und breite Absorption um 427 nm (log ε = 3.3).

### Charakterisierung von Salmycin C:

a) ESI-Massenspektrometrie (electron spray ionisation):
   Molekülionen-Peak von M + H⁺ : 1024.4 Dalton
   Molekülionen-Peak von M + H⁺ + H₂O: 1042.4 Dalton
   Formel: C₄₀H₆₇FeN₆O₂₁
b) ¹H-NMR: Signal bei 2,8 ppm in D₂O (N-Methyl-Gruppe), keine weiteren Signale für Methyl-Protonen.
c) UV-vis der wäßrigen Lösung (Phosphat-Puffer, pH 7,0):
   Endabsorption und breite Absorption um 430 nm (log ε = 3.3).

### Beispiel 9: Gewinnung des Desferridanoxamin-Kaliumsalzes

100 mg Salmycin A werden in 20 ml Wasser gelöst und der pH-Wert mit 0,1N Kalilauge tropfenweise auf pH 9,5 eingestellt. Nach 2 Stunden ist die Hydrolyse beendet. Dann setzt man zur Entfernung des gebundenen Eisens aus dem gebildeten Danoxamin 100 mg Ethylendiamin-tetraessigsäure-di-Kaliumsalz hinzu und läßt weitere 2 Stunden stehen. Hierbei wird die anfänglich lachsfarbene Lösung entfärbt. Die Abtrennung und Reinigung des Desferridanoxamin-Kaliumsalzes erfolgt durch Umkehrphasenchromatographie. Es wird eine Säule von 25 cm Höhe und einem inneren Durchmesser von 3,2 cm verwendet, die mit 200 ml C₁₈-Kieselgel, z.B. ®Nucleosil 12 C₁₈ AB (Macherey & Nagel, Düren, BRD), gefüllt ist. Als Fließmittel wird ein Gemisch aus Wasser und Acetonitril mit einem Konzentrationsgradienten von 0-15 Vol.-% Acetonitril eingesetzt. Die produkthaltigen Fraktionen werden vereinigt und gefriergetrocknet. Es werden 48 mg eines hellbeigen Pulvers gewonnen. Das Produkt hat eine Reinheit von 98%.

### Beispiel 10: Gewinnung des Desferri-Nordanoxamin-Natriumsalzes

100 mg Salmycin C werden entsprechend Beispiel 9 mit 0,1 N Natronlauge hydrolysiert und mit Ethylendiamin-tetraessigsäure-di-Natriumsalz behandelt. Nach anschließender Reinigung resultieren 45 mg Desferri-Nordanoxamin-Natriumsalz in 97 %-iger Reinheit.

### Beispiel 11: Gewinnung von Desferri-Salmycin A, B, C oder D

100 mg Salmycin A, B, C oder D werden in Phosphatpuffer, pH 7, gelöst. Dann setzt man zur Entfernung des gebundenen Eisens 100 mg Ethylendiamin-tetraessigsäure-di-Kaliumsalz hinzu und läßt 2 Stunden stehen. Hierbei wird die anfänglich lachsfarbene Lösung entfärbt. Die Abtrennung und Reinigung des Desferri-Salmycins erfolgt durch Umkehrphasenchromatographie. Es wird eine Säule von 25 cm Höhe und einem inneren Durchmesser von 3,2 cm verwendet, die mit 200 ml C₁₈-Kieselgel, z.B. ®Nucleosil 12 C₁₈ AB (Macherey & Nagel, Düren, BRD), gefüllt ist. Als Fließmittel wird ein Gemisch aus Wasser und Acetonitril mit einem Konzentrationsgradienten von 0-15 Vol.-% Acetonitril eingesetzt. Die produkthaltigen Fraktionen werden vereinigt und gefriergetrocknet. Es wird ein hellbeiges Pulver gewonnen. Das Produkt hat eine hohe Reinheit.

## Patentansprüche

1. Verbindung mit der Summenformel C₂₆H₄₄FeN₅O₁₁ mit einem Molekulargewicht von 658 g/mol, genannt Nordanoxamin, sowie dessen eisenfreie Form mit der Summenformel C₂₆H₄₇N₅O₁₁ und einem Molekulargewicht 605 g/mol, genannt Desferri-Nordanoxamin; Verbindungen der Summenformel C₄₁H₇₃N₇O₂₁, genannt Desferri-Salmycin A, Molekulargewicht 1000 g/mol, der Summenformel C₄₁H₇₂N₆O₂₁, genannt Desferri-Salmycin B, Molekulargewicht 985 g/mol, der Summenformel C₄₀H₇₀N₆O₂₁, genannt Desferri-Salmycin C, Molekulargewicht 971 g/mol und der Summenformel C₄₀H₇₁N₇O₂₁ genannte Desferri-Salmycin D, Molekulargewicht 986 g/mol.

2. Verfahren zur Herstellung von Nordanoxamin gemäß Anspruch 1 durch Hydrolyse von Salmycin C oder D.

3. Verfahren zur Herstellung von Danoxamin mit der Summenformel C₂₇H₄₆FeN₅O₁₁ und einem Molekulargewicht von 672 g/mol durch Hydrolyse von Salmycin A und/oder B.

4. Verfahren zur Herstellung von Desferri-Nordanoxamin gemäß Anspruch 1 aus Salmycin C oder D durch Hydrolyse und Behandlung mit komplexierenden oder eisenbindenen Reagenzien oder durch Reduktion des gebundenen Fe³⁺-Ions.

5. Verfahren zur Herstellung von Desferri-Danoxamin aus Salmycin A und/oder B durch Hydrolyse und Behandlung mit komplexierenden oder eisenbindenen Reagenzien oder durch Reduktion des gebundenen Fe³⁺-Ions.

6. Verfahren zur Herstellung von Desferri-Salmycin A, Desferri-Salmycin B, Desferri-Salmycin C und Desferri-Salmycin D gemäß Anspruch 1 durch Behandlung von Salmycin A, B, C oder D mit komplexierenden oder eisenbindenen Reagenzien oder durch Reduktion des gebundenen Fe³⁺-Ions.

7. Arzneimittel enthaltend eine oder mehrere der Verbindungen Desferri-Nordanoxamin, Desferri-Danoxamin, Desferri-Salmycin A, Desferri-Salmycin B, Desferri-Salmycin C und Desferri-Salmycin D.

8. Verwendung der Verbindungen Desferri-Nordanoxamin, Desferri-Danoxamin, Desferri-Salmycin A, Desferri-Salmycin B, Desferri-Salmycin C oder Desferri-Salmycin D als Chelatoren, bevorzugt zur Bindung der Metallionen Fe³⁺ und Al³⁺.

9. Verwendung der Verbindungen Desferri-Nordanoxamin, Desferri-Danoxamin, Desferri-Salmycin A, Desferri-Salmycin B, Desferri-Salmycin C oder Desferri-Salmycin D und deren physiologisch verträgliche Salze zur Anwendung als Arzneimittel.

10. Verwendung der Verbindungen Desferri-Nordanoxamin, Desferri-Danoxamin, Desferri-Salmycin A, Desferri-Salmycin B, Desferri-Salmycin C oder Desferri-Salmycin D und deren physiologisch verträgliche Salze zur Herstellung von Arzneimitteln zur Behandlung von Hämochromatose oder Alzheimerscher Krankheit.
